# EUROPEAN PATENT APPLICATION

(11) **EP 4 768 025 A1**
(43) Date of publication of application: **01.07.2026**
(21) Application number: 24307294.9
(22) Date of filing: 24.12.2024
(51) Int. Cl.: A61K 31/34, A61P 29/00

(54) **ISOSORBIDE FOR USE IN THE TREATMENT OF ORAL INFLAMMATORY CONDITIONS**

(71) Applicant: ROQUETTE FRERES, 62136 Lestrem (FR)
(72) Inventor: MENTINK, Léon, 59000 LILLE (FR); WILS, Daniel, 59190 MORBECQUE (FR)
(74) Representative: Roquette IP International

(57) **Abstract**

The present disclosure relates to 1,4:3,6-dianhydrohexitol, specifically isosorbide, and compositions comprising 1,4:3,6-dianhydrohexitol for treating oral inflammatory conditions. These conditions, which include both oral mucosal inflammation and periodontal diseases, are characterized by elevated levels of pro-inflammatory mediators, particularly IL-1 alpha and IL-8. Using the EpiOral^{™} model, isosorbide has demonstrated significant ability to reduce IL-1 alpha secretion and IL-8 gene expression in inflamed oral epithelial cells. These results support its therapeutic relevance in treating oral inflammatory conditions through modulation of key inflammatory mediators.

## Description

### TECHNICAL FIELD

The present disclosure relates to compounds and compositions that through their ability to reduce both secretion and expression of interleukins IL-1 and IL-8, are effective for treating oral inflammatory conditions.

### BACKGROUND ART

Oral inflammatory conditions encompass various pathological states affecting different structures within the oral cavity. These conditions primarily include oral mucosal inflammation, which affects the lining of the oral cavity, and periodontal diseases, which involve the tooth-supporting tissues. While distinct in their anatomical locations, both conditions share common inflammatory pathways, particularly involving pro-inflammatory mediators such as IL-1 and IL-8.

Oral mucosal inflammation represents a significant pathological condition affecting the lining of the oral cavity. This inflammation can manifest in various forms and locations within the mouth, including the cheeks, palate, floor of the mouth, and gums. The condition is characterized by redness, swelling, and sometimes ulceration of the oral mucosa, leading to significant discomfort and pain for patients. Various factors can trigger such inflammation, including local irritants, systemic diseases, allergic reactions, or autoimmune conditions. The impact on patients' quality of life can be substantial, affecting basic daily activities such as eating, drinking, and speaking. The complex nature of oral mucosal inflammation often requires targeted therapeutic approaches that can effectively address both the symptoms and underlying inflammatory mechanisms.

Periodontal diseases represent another significant group of oral inflammatory conditions affecting the supporting tissues of the teeth, including the gums, periodontal ligament, and alveolar bone. Gingivitis, the initial stage of these diseases, is characterized by gum inflammation, causing redness, swelling, and bleeding. If left untreated, it can progress to periodontitis, a more severe form that leads to progressive destruction of tooth-supporting tissues and can result in tooth loss. These diseases are particularly debilitating, affecting not only oral health but also the overall quality of life of patients. They can cause pain, difficulties in eating and speaking, persistent bad breath, and have significant aesthetic repercussions, which can lead to lowered self-esteem and psychosocial problems. Moreover, periodontal diseases have been associated with various systemic conditions such as cardiovascular diseases, diabetes, and certain pregnancy complications, highlighting their potential impact on general health.

It is crucial to note that periodontal diseases are not limited to humans but also significantly affect non-human animals and notably pets. For example, it is estimated that over 87% of dogs and 70% of cats over three years of age are affected by periodontal diseases. If left untreated, these conditions can cause multiple problems in the oral cavity and may even lead to damage to internal organs in some pets as they age.

Both oral mucosal inflammation and periodontal diseases require comprehensive treatment approaches.

The management of oral mucosal inflammation relies heavily on conventional therapeutic approaches. Healthcare professionals typically prescribe topical corticosteroids, with triamcinolone acetonide being a common choice. While these medications can effectively reduce inflammation, long-term use raises concerns about mucosal atrophy. Patients are often prescribed protective oral gels and coatings, but these only provide temporary relief without addressing the underlying condition. Similarly, analgesic mouthwashes offer brief pain management but fail to target the root cause. In severe cases, systemic anti-inflammatory medications become necessary, despite their potential for significant side effects.

For periodontal diseases, current protocols include rigorous daily oral hygiene practices, professional mechanical cleaning through scaling, and the use of local antiseptics such as chlorhexidine mouthwashes. More severe cases may necessitate local or systemic antibiotic therapy, and in advanced stages, periodontal surgery including flap procedures or guided tissue regeneration becomes necessary.

These conditions share a common therapeutic challenge: current treatments primarily focus on symptom management and bacterial control through professional interventions and antiseptic agents like chlorhexidine, which can lead to side effects such as tooth discoloration and taste alterations. While necessary, these approaches fail to address the fundamental pathological process. Indeed, chronic inflammation, mediated by pro-inflammatory cytokines such as IL-1 and IL-8, plays a central role in the pathogenesis and progression of oral inflammatory conditions. The lack of treatments specifically targeting these inflammatory mediators limits the long-term efficacy of current therapeutic strategies, often resulting in disease progression despite repeated interventions

Given these significant limitations, particularly regarding inflammation control, there is a need to develop new therapeutic approaches that specifically target inflammatory mediators involved in the pathogenesis of periodontal diseases.

### SUMMARY

The inventors discovered that isosorbide exhibits significant anti-inflammatory properties by reducing expression levels of interleukins IL-1 and IL-8 and thus making it effective for treating oral inflammatory conditions such as oral mucosa inflammation and periodontal diseases.

Isosorbide was tested using two protocols designed to mimic different application scenarios (protocol 1 and 2) on the EpiOral^{™} RHE model, which is a highly differentiated, multilayered model of human buccal epithelial cells. It closely mimics *in vivo* oral tissue characteristics, making it a relevant and reproducible tool for addressing a key mechanism in oral inflammatory conditions. In protocol 1, the secretion of IL-1a, and the expression of IL-8 gene were measured in the presence or in the absence of SLS. In protocol 2 the secretion of IL-1a was measured also in the presence or in the absence of SLS. SLS induces inflammation by disrupting the epithelial barrier and hence represents a plausible approach to mimic inflammation of oral inflammatory conditions.

Protocol 1, which simulates a non-rinsed, prolonged application similar to overnight treatments, assessed the anti-inflammatory effect of the isosorbide in inflamed tissues. Using SLS-induced inflammation as control value, the compound demonstrated significant anti-inflammatory properties by reducing both IL-1α secretion and IL-8 gene expression.

Protocol 2 simulated a rinsed application, similar to a mouthwash use. Using the same inflammatory model where SLS induces inflammation (control value), the isosorbide showed significant anti-inflammatory properties by reducing IL-1α secretion.

Hence, isosorbide effectively reduces inflammation induced by SLS in both prolonged and rinsed application scenarios.

In oral mucosal inflammation, cytokines play a central role (Neurath, Nicole, et Marco Kesting. « Cytokines in Gingivitis and Periodontitis: From Pathogenesis to Therapeutic Targets ». Frontiers in Immunology 15 (2024): 1435054; Najmi et al. 2023 "Oral inflammation and the role of cytokine", Jurnal eduhealth, vol. 14, no 02, E-ISSN. 2808-4608). Interleukines like IL-1 and IL-8 are part of the cytokines that play pivotal roles in the inflammatory cascade.

IL-1 acts as a primary mediator initiating the inflammatory response in oral mucosa by promoting the recruitment of immune cells and stimulating the production of other inflammatory mediators. This cytokine also induces the expression of adhesion molecules on endothelial cells, facilitating leucocyte infiltration into the inflamed tissue.

IL-8 further amplifies the inflammatory response by specifically attracting neutrophils to the site of inflammation in the oral mucosa. Moreover, IL-8 stimulates angiogenesis and promotes the release of matrix metalloproteinases, contributing to tissue remodelling. The persistent elevation of these cytokines in oral mucosal tissues maintains a chronic inflammatory state, leading to continued tissue damage and delayed healing.

Periodontal diseases, such as gingivitis and periodontitis, involve chronic inflammation in the oral mucosa. Gingivitis can progress to periodontitis, with both conditions activating immune responses that produce pro-inflammatory cytokines like IL-1 and IL-8. IL-1 has been identified as a major mediator of tissue destruction in periodontal disease.

Genetic studies link IL-1 polymorphisms to increased periodontitis risk, and successful treatments often reduce IL-1 levels. IL-8 also plays a crucial role in inflammation, although its timing in secretion may vary. Both cytokines are important targets for understanding and treating periodontal diseases.

Hence, the demonstrated ability of isosorbide to reduce both IL-1 and IL-8 expression makes it particularly suitable for treating oral inflammatory condition such as oral mucosal inflammation and periodontal diseases, where these inflammatory mediators play a crucial role in disease progression.

Hence, the present invention relates to 1,4:3,6-dianhydrohexitols and preferably isosorbide for use in reducing expression levels of interleukins in a subject.

The present invention also relates to1,4:3,6-dianhydrohexitols and preferably isosorbide for use in the treatment of oral inflammatory conditions such as oral mucosa inflammation and periodontal disorders.

### BRIEF DESCRIPTION OF THE DRAWINGS

**FIGURE 1****:** Summary of protocol 1.
**FIGURE 2****:** Summary of protocol 2.

### DETAILED DESCRIPTION

The present invention relates to 1,4:3,6-dianhydrohexitols and preferably isosorbide for use in the treatment of oral inflammatory conditions.

### 1,4:3,6-dianhydrohexitols

1,4:3,6-dianhydrohexitols, also known simply as dianhydrohexitols or isohexides, are products of double internal dehydration of C6 polyols (hexitols) such as sorbitol, mannitol, and iditol. In this application, the term "1,4:3,6-dianhydrohexitol" encompasses isosorbide (1,4:3,6-dianhydrosorbitol), isomannide (1,4:3,6-dianhydromannitol), and isoidide (1,4:3,6-dianhydroiditol).

According to one embodiment, the 1,4:3,6-dianhydrohexitol is isosorbide.

### Oral inflammatory conditions

Oral inflammatory conditions represent a group of disorders affecting the oral cavity, including both oral mucosal inflammation and periodontal diseases. Despite their distinct anatomical locations, these conditions share common inflammatory pathways mediated predominantly by IL-1 and IL-8, which are central to tissue inflammation and disease progression.

In an embodiment, the present invention relates to 1,4:3,6-dianhydrohexitols and preferably isosorbide for use in the treatment of oral inflammatory conditions in a subject in need thereof having an increased expression level of IL-1 and IL-8 in oral tissue.

### Periodontal disorders

Periodontal diseases are a group of inflammatory conditions affecting the supporting tissues of the teeth, including the gums, periodontal ligament, and alveolar bone. Gingivitis, the initial stage of these diseases, is characterized by gum inflammation, causing redness, swelling, and bleeding. If left untreated, it can progress to periodontitis, a more severe form that leads to progressive destruction of tooth-supporting tissues and can result in tooth loss.

In an embodiment, the periodontal disorder is selected among gingivitis and periodontitis.

In an embodiment, the periodontal disorder is gingivitis.

In an embodiment, the periodontal disorder is periodontitis.

Periodontal diseases, including gingivitis and periodontitis, are characterized by chronic inflammatory processes in the oral mucosa. Gingivitis precludes to periodontitis. These conditions are primarily caused by microflora and microbial biofilms, which activate both the innate and adaptive immune systems, leading to the production of pro-inflammatory cytokines (Neurath, Nicole, et Marco Kesting. « Cytokines in Gingivitis and Periodontitis: From Pathogenesis to Therapeutic Targets ». Frontiers in Immunology 15 (2024): 1435054.). Among these cytokines, interleukin-1 (IL-1) and interleukin-8 (IL-8) play crucial roles in the pathogenesis of gingivitis and periodontitis.

IL-1, has been identified as a major mediator of tissue destruction in periodontal disease (Seymour, Gregory J., et Erica Gemmell. « Cytokines in Periodontal Disease: Where to from Here? » Acta Odontologica Scandinavica 59, no 3 (2009): 167 73). IL-1 alpha is a precursor of IL-1 beta, and is sometimes easier to quantify than IL-1 beta.

Studies have shown significantly increased levels of IL-1β in periodontal tissues and gingival fluid from diseased sites compared to healthy sites. IL-1β is a powerful and potent bone-resorbing cytokine that up-regulates matrix metalloproteinases and down-regulates tissue inhibitors of metalloproteinase production, suggesting a key role in degrading the extracellular matrix in periodontitis (Orozco, A., E. Gemmell, M. Bickel, et G. J. Seymour. « Interleukin-1beta, Interleukin-12 and Interleukin-18 Levels in Gingival Fluid and Serum of Patients with Gingivitis and Periodontitis ». Oral Microbiology and Immunology 21, n° 4 (2006): 256-60).

The importance of IL-1 in periodontal disease is further supported by genetic studies. IL-1 gene polymorphisms (e.g., coding variants for Lys3, Asn3, and Met 256) have been correlated with an increased risk for the development of periodontitis. Moreover, in patients with periodontitis, a significant decrease in levels of IL-1β was observed after non-surgical periodontal treatment, suggesting that successful therapy prevents disease progression at least in part by suppression of IL-1 function (Neurath, Nicole, et Marco Kesting. « Cytokines in Gingivitis and Periodontitis: From Pathogenesis to Therapeutic Targets ». Frontiers in Immunology 15 (2024): 1435054.).

Kinane and co-authors published a possible diagnosis of gingivitis by the measurement of IL-1 in the saliva or in the crevicular fluid (Kinane, D. F., F. P. Winstanley, E. Adonogianaki, et N. A. Moughal. "Bioassay of interleukin 1 (IL-1) in human gingival crevicular fluid during experimental gingivitis", Archives of Oral Biology, vol. 37, Issue 2, February 1992, pages 153-56).

IL-8, another key pro-inflammatory cytokine, has also been implicated in the pathogenesis of periodontal diseases. Polepalle and co-workers identified IL-8 among the cytokines triggering the acute phase of inflammation and having strong proinflammatory properties in the context of periodontal diseases (Tejaswin Polepalle, Srinivas Moogala, Shalini Boggarapu, Divya Sai Pesala, Firoz Babu Palagi, "Acute Phase Proteins and Their Role in Periodontitis: A Review", Journal Of Clinical And Diagnostic Research, 2015 Nov, vol-9(11): ZE01-ZE05). These include TNF-α/β, IL-6, IFN-α/γ, macrophage inhibitory protein-1 and IL1α/ β and IL-8.

Furthermore, according to Boronat-Catala et al. there is a correlation between the saliva concentration of IL-1a/b and the early stage of gingivitis. The results are a bit controversy for IL-8 but they are explained by the timing of the trial estimated too early for IL-8. Effectively, IL-8 is secreted a bit after IL-1 (Boronat-Catala, M., M. Catala-Pizarro, et Jv. Bagan. « Salivary and Crevicular Fluid Interleukins in Gingivitis ». Journal of Clinical and Experimental Dentistry, 2014, vol.6(2), p.175-179).

In an embodiment, periodontal disorders are inflammation-predominant periodontal disorders.

Plaque-induced gingivitis represents a condition where the primary pathological driver is the accumulation of dental bacterial biofilm. In this form, the inflammatory response manifests as a direct consequence of bacterial presence, leading to gingival inflammation. The therapeutic approach primarily focuses on plaque elimination through mechanical control and antiseptic treatments, as removing the bacterial trigger is considered key to resolving the condition.

In contrast, inflammation-predominant gingivitis is characterized by an inflammatory response that can be influenced or exacerbated by various non-bacterial factors, including stress, hormonal changes, and certain medications. In this form, the inflammatory response may be disproportionate to the bacterial load present, with inflammatory mediators such as IL-1 and IL-8 playing a central role in the pathological process. This form might particularly benefit from targeted anti-inflammatory therapeutic approaches.

Hence, in an embodiment, the gingivitis is an inflammation-predominant gingivitis.

Plaque-induced periodontitis primarily develops as a progression of untreated gingivitis, where bacterial biofilm accumulation leads to the destruction of tooth-supporting tissues. In this form, the pathological process is initiated and driven by bacterial colonization, with a particular emphasis on specific periodontal pathogens. The disease progression correlates strongly with the presence and composition of the subgingival biofilm, and traditional therapeutic approaches focus on bacterial control through mechanical debridement and antimicrobial strategies.

In contrast, inflammation-predominant periodontitis is characterized by an excessive or dysregulated host inflammatory response that may be disproportionate to the bacterial challenge. In these cases, while bacteria are present, the tissue destruction is primarily driven by an aberrant inflammatory response, with elevated levels of pro-inflammatory mediators such as IL-1 and IL-8 playing a crucial role in tissue degradation. This form may be particularly associated with systemic conditions that modify host response, such as diabetes or immune disorders, and might benefit more from therapeutic approaches targeting the inflammatory component.

Hence, in an embodiment, the periodontitis is an inflammation-predominant periodontitis.

### Oral mucosa inflammation

Oral mucosa inflammation is a pathological condition affecting the lining tissues of the oral cavity, characterized by an inflammatory response in the oral mucous membranes. This condition can manifest in various locations including the cheeks, palate, floor of the mouth, and non-gingival areas of the oral cavity. The inflammatory process is characterized by typical signs of inflammation such as redness, swelling, and sometimes ulceration, leading to pain and discomfort. The condition can be triggered by various factors including physical irritants, chemical agents, allergic reactions, or systemic conditions. At the molecular level, the inflammatory response involves the release of pro-inflammatory mediators, particularly IL-1 and IL-8, which play key roles in initiating and maintaining the inflammatory process in the affected tissues.

As used herein, the terms "treatment", "treat" or "treating" refer to all therapeutic approaches for oral inflammatory conditions, whether oral mucosal inflammation or periodontal diseases. These approaches aim either to reduce existing symptoms or to prevent their onset or worsening.

In oral inflammatory conditions, inflammation can become chronic or excessive. This persistent inflammation is characterized particularly by the overproduction of inflammatory mediators such as IL-1 and IL-8. The treatment therefore aims to reduce the production of these inflammatory mediators to decrease inflammation in oral tissues.

### Dose

In an embodiment, the isosorbide for use is administered to a subject at a dose comprised between 0.1 and 50 g per day.

The exact dosage can be determined by one skilled in the art, taking into account various factors such as the specific oral inflammatory condition being treated, the severity of the condition, and the individual characteristics of the patient, including age, weight, and overall health.

### Administration

In an embodiment, the isosorbide for use is administered to a subject orally.

In an embodiment, isosorbide is administered to a subject orally, meaning it is taken through the mouth and absorbed via the digestive system to exert its therapeutic effects.

In an embodiment, the isosorbide for use is administered to a subject topically.

In an embodiment, isosorbide is administered to a subject topically, meaning it is applied directly to a specific area of the body, such as the skin or mucous membranes, for localized therapeutic effects.

### Reducing secretion and expression of interleukins

The present invention also relates to isosorbide for use in the treatment of oral inflammatory conditions, wherein said oral inflammatory conditions are characterized by increased expression levels of IL-1 and IL-8.

In an embodiment, oral mucosa inflammation is characterized by an increased expression level of IL-1 and IL-8 in oral tissue.

In an embodiment, gingivitis and periodontitis are characterized by an increased expression level of IL-1 and IL-8 in oral tissue.

Increased expression levels of IL-1 and IL-8 refer to the elevated production and presence of the interleukin-1 (IL-1) and interleukin-8 (IL-8) proteins or their corresponding mRNA in a biological sample compared to a control value. The control value represents the baseline or normal levels of IL-1 and IL-8 expression in a healthy population or in a non-inflamed tissue. An increase in expression levels is typically quantified by comparing the amount of IL-1 and IL-8 in the sample to this control value, indicating a statistically significant elevation. This elevation suggests an enhanced inflammatory response, which can be indicative of an underlying inflammatory condition or disease.

The present invention also relates to isosorbide for use in reducing expression level of interleukins in a subject.

In an embodiment, the present invention relates to isosorbide for use in reducing expression level of interleukins in oral tissues of a subject.

Oral tissues encompass the complex array of specialized tissues within the oral cavity, comprising epithelial and connective tissues of the oral mucosa, periodontal structures (including gingiva, periodontal ligament, and alveolar bone), salivary glands, and associated vasculature and nerve supplies.

In a preferred embodiment, the oral tissues are chosen among epithelial tissue and periodontal structures.

In a preferred embodiment, interleukins are IL-1 and IL-8.

When referring to "IL-1 and IL-8 expression level", it is referred herein either to, the expression level of the gene (such as mRNA expression) and/or to the corresponding protein expression.

In a preferred embodiment, the present invention relates to isosorbide for use in reducing IL-1 protein expression and IL-8 gene expression levels of interleukins in oral tissues of a subject.

Expression of interleukins such as IL-1 and IL-8 can be quantified by determining gene or protein expression of IL-1 and IL-8 in the oral tissue of a subject or in an *ex vivo* model.

The quantification may be relative (by comparing the amount of interleukins expression level to a control with known amount of interleukins amount for example and detecting "higher" or "lower" amount compared to that control) or more precise, at least to determine the specific amount relative to a known control amount.

Increased expression levels of IL-1 and IL-8 refer to the elevated production and presence of the interleukin-1 (IL-1) and interleukin-8 (IL-8) proteins or their corresponding mRNA in a biological sample compared to a control value. The control value represents the baseline or normal levels of IL-1 and IL-8 expression in a healthy population or in a non-inflamed tissue. An increase in expression levels is typically quantified by comparing the amount of IL-1 and IL-8 in the sample to this control value, indicating a statistically significant elevation. This elevation suggests an enhanced inflammatory response, which can be indicative of an underlying inflammatory condition or disease.

"Gene expression", "gene product" or "expression" are all used herein interchangeably and refer to the nucleic acids or amino acids (e.g., peptide or polypeptide) generated when a gene is transcribed and translated. In a particular embodiment "expression level" "gene expression", "gene product" or "expression" denotes the amount of nucleic acid (e.g. mRNA) or protein generated when a gene is transcribed and translated in a cell.

The expression level of interleukins genes may be determined by any suitable methods known by skilled persons.

For example, the nucleic acid contained in the sample is first extracted according to standard methods, for example using lytic enzymes or chemical solutions or extracted by nucleic-acid-binding resins following the manufacturer's instructions. The extracted mRNA is then detected by hybridization (e.g., Northern blot analysis, DNA microarray) and/or amplification (e.g., RT-PCR) or by direct RNA sequencing.

The expression level of interleukins protein may also be determined by any suitable methods known by skilled persons. The quantity of the protein may be measured, for example, by semiquantitative Western blots, enzyme-labelled and mediated immunoassays, such as ELISAs, biotin/avidin type assays, radioimmunoassay, immunoelectrophoresis, mass spectrometry or immunoprecipitation or by protein or antibody arrays. For example, interleukins ELISA kits are commercially available.

In a particular embodiment, the subject is previously identified as having a higher expression level of interleukins in a biological sample (e.g., patient's inflammatory tissue) as compared to a control value (e.g., healthy tissue) by: a) determining interleukins expression level in a patient biological sample and b) comparing the interleukins expression level value to a control value.

In a particular embodiment, an increased level means a statistically significant increase of such expression level of interleukins in patient sample (e.g., inflammatory tissue) as compared to a control value (e.g., healthy tissue), preferably, at least 1.2; preferably 1.5; 1.75; 2; 2.2; more preferably at least 2.5-fold increase of the control value.

According to the present disclosure, the terms "threshold value", "control value" or "cut-off value" can be used interchangeably and can be determined experimentally, empirically, or theoretically. The control value refers to interleukins' expression level in a biological sample obtained from a general population or from a selected population of subjects. For example, the general population may comprise apparently healthy subjects, such as individuals who have not previously had any sign or symptoms indicating the presence of an oral inflammatory conditions as described above. The term "healthy subjects" as used herein refers to a population of subjects who do not suffer from any known condition, and in particular, who are not affected with a disease, preferably any oral inflammatory conditions as described above. According to a preferred embodiment of the disclosure, said control value is the level of interleukins expression in the healthy subject tissue corresponding to the patient's inflammatory tissue.

The control value may alternatively be a predetermined value such as a threshold value, a standard value or a range obtained from other source than the patient's data. In particular embodiment, the control value refers to interleukins' expression level in a biological sample obtained from other source than the patient's data, for example patients who are known to have low interleukins expression level. The control value may be established based upon comparative measurements between general population or patients who are known to have low interleukins expression level and patients with high interleukins expression level and calculating the statistical significance between interleukins expression level of a collection of patient samples. Said predetermined reference value (cut-off value) can consist of the value for which the highest statistical significance is calculated. The comparison step may be carried out manually or computer assisted.

### Compositions

In an embodiment, the topical composition is chosen among mouthwash, toothpaste, gel, foam, ointment, cream, solution, spray, powder, strips, wipes, oral rinse, chewing gum, lozenge, varnish, oral patch, bioadhesive gel.

In another embodiment, the oral composition is chosen among tablets, capsules, liquid solutions, suspensions, syrups, lozenges, chewable tablets, orally disintegrating tablets, sublingual tablets, buccal tablets, effervescent tablets, granules.

In an embodiment, the composition comprises from 0.1 to 50% by weight, preferably from 0.1 to 30% by weight, preferably from 0.2 to 15% by weight, preferably from 0.5 to 7% by weight, based on the total weight of the composition, of isosorbide.

In an embodiment, the composition comprises another therapeutic agent chosen among preservatives like bacteriostatic or bactericidal compounds. For example, preservatives like chlorhexidine, zinc sales, ethanol, and cetylpyridinium chloride.

### Subjects

In an embodiment, the subject is chosen among a human and a non-human animal.

In an embodiment, the subject is a human.

In another embodiment, the subject is a non-human animal chosen among pets, in particular among a cat or a dog

The present invention also relates to a method treating oral inflammatory conditions such as oral mucosa inflammation and periodontal disorders comprising administering an effective amount of isosorbide to a subject in need thereof.

The present invention also relates to a pharmaceutical composition comprising isosorbide for use in the treatment of oral inflammatory conditions such as oral mucosa inflammation and periodontal disorders.

The present invention also relates to the use of isosorbide in the manufacture of a medicament for the treatment of oral inflammatory conditions such as oral mucosa inflammation and periodontal disorders.

An effective amount of isosorbide to a subject in need thereof refers to a quantity of isosorbide that is sufficient to achieve the desired therapeutic effect in treating the condition or disorder in the subject. This amount can vary depending on factors such as the specific condition being treated, the severity of the condition, the subject's age, weight, overall health, and individual response to the treatment. The effective amount is determined by one skilled in the art through clinical experience, empirical data, and standard medical practices to ensure both efficacy and safety for the subject.

All the previously disclosed embodiments are encompassed within these aspects.

The invention will be further illustrated by the following figures and examples. However, these examples and figures should not be interpreted in any way as limiting the scope of the present invention.

### Embodiments of the invention

Embodiment 1: 1,4:3,6-dianhydrohexitol for use in the treatment of oral inflammatory conditions.

Embodiment 2: 1,4:3,6-dianhydrohexitol for use according to embodiment 2, wherein the oral inflammatory conditions is selected among oral mucosa inflammation and periodontal disorders.

Embodiment 3: 1,4:3,6-dianhydrohexitol for use according to embodiment 3, wherein the periodontal disorder is selected among gingivitis and periodontitis, preferably is periodontitis.

Embodiment 4: 1,4:3,6-dianhydrohexitol for use according to anyone of embodiments 1 to 3, wherein said oral inflammatory conditions are characterized by increased expression levels of IL-1 and IL-8.

Embodiment 5: 1,4:3,6-dianhydrohexitol for use according to anyone of embodiments 1 to 4, wherein isosorbide is administered to a subject at a dose comprised between 0.1 and 50 g per day.

Embodiment 6: 1,4:3,6-dianhydrohexitol for use according to anyone of embodiments 1 to 5, wherein isosorbide is administered to a subject orally by an oral composition.

Embodiment 7: 1,4:3,6-dianhydrohexitol for use according to anyone of embodiments 1 to 6, wherein isosorbide is administered to a subject topically by a topical composition.

Embodiment 8: 1,4:3,6-dianhydrohexitol for use according to embodiment 7, wherein the topical composition is chosen among mouthwash, toothpaste, gel, foam, ointment, cream, solution, spray, powder, strips, wipes, oral rinse, chewing gum, lozenge, varnish, oral patch, bioadhesive gel.

Embodiment 9: 1,4:3,6-dianhydrohexitol for use according to embodiment 6, wherein the oral composition is chosen among tablets, capsules, liquid solutions, suspensions, syrups, lozenges, chewable tablets, orally disintegrating tablets, sublingual tablets, buccal tablets, effervescent tablets, granules.

Embodiment 10: 1,4:3,6-dianhydrohexitol for use according to anyone of claims 6 to 9, wherein the oral or topical composition comprises from 0.1 to 50% by weight, preferably from 0.1 to 30% by weight, preferably from 0.2 to 15% by weight, preferably from 0.5 to 7% by weight, based on the total weight of the composition, of 1,4:3,6-dianhydrohexitol.

Embodiment 11: 1,4:3,6-dianhydrohexitol for use according to anyone of embodiment 1 to 10, wherein the subject is chosen among a human and a non-human animal.

Embodiment 12: 1,4:3,6-dianhydrohexitol for use according to embodiment 11, wherein the subject is a human.

Embodiment 13: 1,4:3,6-dianhydrohexitol for use according to embodiment 11, wherein the subject is a non-human animal chosen among pets, in particular among a cat or a dog.

Embodiment 14: 1,4:3,6-dianhydrohexitol for use according to any the preceding embodiments, wherein said 1,4:3,6-dianhydrohexitol is isosorbide.

### EXAMPLES

### Example 1: isosorbide anti-inflammatory effect on oral inflammatory condition induced by SLS, using oral mucosal tissue model.

Isosorbide was evaluated on an oral inflammatory condition induced by sodium lauryl sulfate (SLS), using oral mucosal tissue model, with the quantification of cytokine production and of gene expression.

### Materials

The oral mucosa model "EpiOral^{™} RHE" and culture medium were obtained from MatTek^{®}. MatTek's EpiOral^{™} tissues consist of normal, human-derived oral epithelial cells. The cells have been cultured to form multilayered, highly differentiated models of the human buccal phenotypes. The tissues are cultured on specially prepared cell culture inserts using serum free medium and attain levels of differentiation on the cutting edge of *in vitro* cell culture technology. The EpiOral^{™} tissue models exhibit *in vivo-like* morphological and growth characteristics which are uniform and highly reproducible.

EpiOral^{™} expresses cytokeratin K13 in all layers except the most apical ones and weakly express cytokeratin K14 in the upper layers of the tissue. The tissues also produce the naturally occurring antimicrobial peptides called human beta defensins (HBDs). EpiOral^{™} constitutively expresses HBD-1 and HBD-3 but not HBD-2.

A growing body of data indicates that EpiOral^{™} effectively provide an inexpensive, non-animal means to assess oral irritation, toxicology, and pathology-related issues (Bacca, L.A, Jewell-Motz, E.A, "Novel in vitro methodology evaluating potential mucosal irritation of oral care formulations", International Association of Dental Research annual meeting, abstract #1189, Baltimore, MD, March 2005).

Sodium Lauryl Sulfate (SLS) was obtained from Sigma-Aldrich^{®}, and the kit for Cytokines measurement (TNF-alpha and IL-1a) from Millipore^{®}.

Kit HTRF for cytokines measurement were used and bought to CISBIO-Revvity.

The model of SLS application on oral mucosa represents a relevant approach to mimic the initial mechanisms of oral inflammatory conditions such as oral mucosa inflammation and gingivitis and its potential progression towards periodontitis. Indeed, SLS acts as a disruptor of the epithelial barrier, creating irritation and local inflammation that paves the way for subsequent aggression. This model is thus a valuable tool for studying inflammatory mechanisms. This model provides a plausible and effective platform for testing potential therapeutic interventions such as oral mucosa inflammation and periodontal disorders.

Isosorbide, as sold under the tradename "Beauté by Roquette^{®} PO 500" by Roquette, was a liquid containing 80 wt% of isosorbide and 20 %wt of water.

### Methods

### Cell isolation and culture:

Cell Isolation and culture: EpiOral^{™} RHE were prepared by the manufacturer (MatTek^{®}) and stored at +4°C. Once the EpiOral^{™} tissues were removed from the refrigerator, a period of equilibration was done overnight before the experimental protocol.

### Cytokines quantification:

Cytokines (IL-1a) were analysed with a HTRF assay kit (Homogeneous Time Resolved Fluorescence). Kits were purchased from CISBIO- Revvity.

### Statistical evaluation:

The results were evaluated by comparing the variance according to the Student's t-test and results of p value are presented in a separate table. When a significant difference was found, cases were highlighted with two asterisks (**) for p<0,05 and with one asterisk for (*) for p<0,1.

### Protocol 1: non-washed application

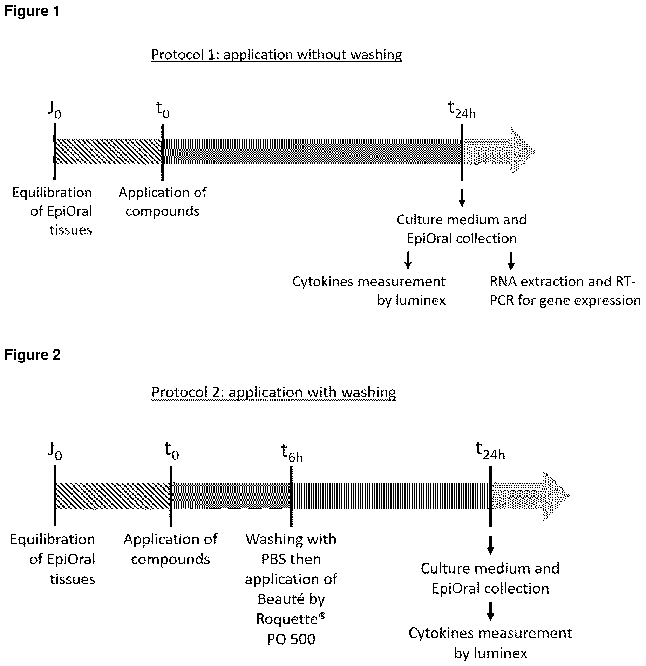

The protocol 1 (figure 1) consisted in the application on the EpiOral^{™} RHE tissues of a compound without any washing after the application of the compound. The compound was thus applied continuously for 24 hours. The compound was chosen from PBS (for control), SLS aqueous solution at 0,2 wt%, Beauté by Roquette^{®} PO 500 at 5,7 wt% in water, or an aqueous solution comprising Beauté by Roquette^{®} PO 500 at 5,7 wt% and SLS at 0,2 wt%.

Protocol 1 simulated a non-rinsed, prolonged application of the tested compound. In this protocol, the compound was applied to the tissues and left in place for 24 hours without rinsing. This method mimics the use of products that are designed for extended contact with oral tissues, such as overnight treatments or long-lasting topical applications. The extended exposure time allowed for the evaluation of the compound' effectiveness over a longer period, as well as any potential cumulative effects or interactions that may occur with prolonged contact.

### Protocol 2: washed application

The protocol 2 (figure 2) consisted in the addition on the EpiOral^{™} RHE tissues of the compound for 6 hours, after which the EpiOral^{™} RHE tissues was washed with PBS (Phosphate Buffered Saline), and then the 5,7 wt% aqueous solution of Beauté by Roquette PO 500 was then applied and left in place for 18 hours. The compound was chosen from PBS (for control), SLS aqueous solution at 0,2 wt%, or an aqueous solution comprising Beauté by Roquette^{®} PO 500 at 5,7 wt% and SLS at 0,2 wt%.

Protocol 2 mimicked a rinsed application, such as a mouthwash, followed by the non-rinsed application of isosorbide. In this protocol, the tissues were exposed to SLS during only 6 hours and then washed with PBS, simulating the process of using and rinsing out a mouthwash containing an irritating ingredient. The Beauté by Roquette PO 500 was then applied for 18 hours, simulating the process of using a non-rinsed mouthwash with this compound. This method allows for the evaluation of the isosorbide's effectiveness in conditions that closely resemble real-world usage of oral care products where the irritating ingredients are in contact with oral tissues for a limited time before being rinsed away.

### Both protocol 1 and 2 are summarized in Figures 1 and 2.

After 24h, Cytokines quantification and Gene expression analysis:
- Cytokines (IL-1a) were analyzed with a milliplex assay kit and measured on the apparatus Luminex MAGPIX.
- The RNAs are extracted by using B-mercaptoethanol lysis buffer and GeneJEt RNA purification kit. Epiroal^{™} tissues samples are gently retrieved using forceps and put in the lysis buffer. The lysate is then mixed and frozen to -8°C. Then EtOH 70% is added (V/V). The chaotropic salt and ethanol cause RNA to bind to the silica membrane while the lysate is spun through the column. Subsequently, impurities are effectively removed from the membrane by washing the column with wash buffers.

Pure RNA is then eluted under low ionic strength conditions with nuclease-free water. The reverse transcription was then carried out with 1µg of RNA with the Maxima first strand synthesis kit. qPCR were realized on VIA7 apparatus (Applied Biosystems). For qPCR, primers has been designed by ambiotis.

For calculating relative quantification of target gene mRNA expression, we will use the following procedure.
> Δ**Ct gene of interests CtSample- CtVehicle**
> Δ**Ctβ-house keeping gene= CtSample-CtVehicle**

Then, ΔΔCt represented the difference between ΔCt house keeping gene and ΔCt gene of interest.
➢ ΔΔ**Ct**=Δ**Ctβhouse keeping gene -ACt gene of interest**

Finally, the N-fold differential expression of gene of interest mRNA samples compared to the vehicle will be express as 2ΔΔCt.

The results are expressed in fold induction compared to the control and are obtained by using the ΔΔCt method where the Ct correspond to the number of cycles necessary to generate a fluorescent signal above the predefined threshold (the more you have of targets, the less cycles you require to reach this threshold).

### Results

Protocol 1:
- *Cytokines quantification: Secretion of IL-1a*

**Table 1:**

| **PROTOCOL 1** | **Average secretion of IL-1a (pg/mL)** | **Significant vs SLS 0,2%** |
|---|---|---|
| **Control** | 186,01 | ** p< 0,05 |
| **5,7% Beauté by Roquette^{®} PO 500(Isosorbide)** | 240,00 | ** p< 0,05 |
| **0,2% SLS** | 5153,59 | - |
| **5,7% Beauté by Roquette^{®} PO 500 (Isosorbide)** + **0,2% SLS** | 2995,79 | ** p< 0,05 |

Table 1 presents the results of IL-1a secretion in EpiOral^{™} RHE tissues under different treatments in Protocol 1.

**Control:** The average IL-1a secretion is 186.01. This group serves as a reference for comparing other treatments.

**5.7% Beauté by Roquette^{®} PO 500 (Isosorbide):** The average IL-1a secretion is 240.00. This treatment does not show a significant difference compared to the control. Hence, isosorbide does not increase IL-1a secretion and thus does not induce inflammation when applied on EpiOral^{™} RHE tissues.

**0.2% SLS:** The average IL-1a secretion is 5153.59. This treatment shows a significant increase in IL-1a, indicating inflammation induced by SLS.

**5.7% Beauté by Roquette@ PO 500 (isosorbide) + 0.2% SLS:** The average IL-1a secretion is 2995.79. This treatment significantly reduces inflammation compared to SLS alone, as indicated by ** p< 0.05.

In summary, isosorbide significantly reduces the inflammation induced by SLS, as shown by the decrease in IL-1a secretion.

### • Gene expression of IL-8 in Epioral Tissues

**Table 2:**

| **PROTOCOL 1** | **Average resu It** | **Significant** |
|---|---|---|
| **Control** | 1,00 | ** p< 0,05 vs SLS 0,2% |
| **5,7% Beauté by Roquette^{®} PO 500** | 1,09 | ** p< 0,05 vs Control |
| **0,2% SLS** | 2,43 | ** p< 0,05 vs Control |
| **5,7% Beauté by Roquette^{®} PO 500 + 0,2% SLS** | 1,15 | ** p< 0,05 vs SLS 0,2% |

Table 2 presents the results of IL-8 gene expression in EpiOral^{™} RHE tissues under different treatments in Protocol 1.

**Control:** The average IL-8 expression is 1.00. This serves as the baseline for comparison.

**5.7% Beauté by Roquette^{®} PO 500 (Isosorbide):** The average IL-8 expression is 1.09 showing no significant difference from the control. Hence, isosorbide does not increase IL-8 secretion and thus does not induce inflammation when applied on EpiOral^{™} RHE tissues.

**0.2% SLS:** The average IL-8 expression is 2.43 indicating an increase in IL-8 expression and thus an inflammation induced by SLS.

**5.7% Beauté by Roquette^{®} PO 500 (isosorbide)** + **0.2% SLS:** The average IL-8 expression is 1.15 significantly reduced compared to SLS alone, as indicated by ** p< 0.05.

In summary, isosorbide significantly reduces IL-8 expression when combined with SLS, suggesting an anti-inflammatory effect.

### Protocol 2:

- *Cytokines quantification: Secretion of IL-1a*

**Table 3:**

| **PROTOCOL 2** | **Average secretion of IL-1a (pg/mL)** | **Significant vs SLS 0,2%** |
|---|---|---|
| **Control** | 35 | ** p< 0,05 |
| **0,2% SLS** | 609 | - |
| **5,7% Beauté by Roquette^{®} PO 500 + 0,2% SLS** | 441 | ** p< 0,05 |

Table 3 presents the results of IL-1a secretion in EpiOral^{™} RHE tissues under different treatments in Protocol 2.

### Control: Baseline IL-1a secretion.

**0.2% SLS:** Significant increase in IL-1a, indicating inflammation.

**5.7% Beauté by Roquette^{®} PO 500 (Isosorbide)** + **0.2% SLS:** Significant reduction in IL-1a compared to SLS alone, indicating an anti-inflammatory effect.

Isosorbide effectively reduces inflammation induced by SLS, as evidenced by decreased levels of IL-1a and IL-8. This suggests its potential as an anti-inflammatory agent in periodontal treatments.

Results obtained in the EpiOral^{™} model demonstrate that the compound significantly reduces IL-1alpha secretion and IL-8 gene expression in inflamed oral epithelial cells. This ability to modulate these key inflammatory mediators is particularly relevant for the treatment of oral inflammatory conditions, where IL-1alpha and IL-8 play a central role in the inflammatory cascade. Indeed, these interleukins are involved in both oral mucosal inflammation and periodontal diseases, making the compound a promising therapeutic approach for these oral inflammatory conditions.

## Claims

1. 1,4:3,6-dianhydrohexitol for use in the treatment of oral inflammatory conditions.

2. 1,4:3,6-dianhydrohexitol for use according to claim 2, wherein the oral inflammatory conditions are selected among oral mucosa inflammation and periodontal disorders.

3. 1,4:3,6-dianhydrohexitol for use according to claim 3, wherein the periodontal disorder is selected among gingivitis and periodontitis, preferably is periodontitis.

4. 1,4:3,6-dianhydrohexitol for use according to anyone of claims 1 to 3, wherein said oral inflammatory conditions are **characterized by** increased expression levels of IL-1 and IL-8.

5. 1,4:3,6-dianhydrohexitol for use according to anyone of claims 1 to 4, wherein 1,4:3,6-dianhydrohexitol is administered to a subject at a dose comprised between 0.1 and 50 g per day.

6. 1,4:3,6-dianhydrohexitol for use according to anyone of claims 1 to 5, wherein 1,4:3,6-dianhydrohexitol is administered to a subject orally.

7. 1,4:3,6-dianhydrohexitol for use according to anyone of claims 1 to 6, wherein 1,4:3,6-dianhydrohexitol is administered to a subject topically.

8. 1,4:3,6-dianhydrohexitol for use according to claim 7, wherein the topical composition is chosen among mouthwash, toothpaste, gel, foam, ointment, cream, solution, spray, powder, strips, wipes, oral rinse, chewing gum, lozenge, varnish, oral patch, bioadhesive gel.

9. 1,4:3,6-dianhydrohexitol for use according to claim 6, wherein the oral composition is chosen among tablets, capsules, liquid solutions, suspensions, syrups, lozenges, chewable tablets, orally disintegrating tablets, sublingual tablets, buccal tablets, effervescent tablets, granules.

10. 1,4:3,6-dianhydrohexitol for use according to anyone of claims 6 to 9, wherein the oral or topical composition comprises from 0.1 to 50% by weight, preferably from 0.1 to 30% by weight, preferably from 0.2 to 15% by weight, preferably from 0.5 to 7% by weight, based on the total weight of the composition, of 1,4:3,6-dianhydrohexitol.

11. 1,4:3,6-dianhydrohexitol for use according to anyone of claims 1 to 10, wherein the subject is chosen among a human and a non-human animal.

12. 1,4:3,6-dianhydrohexitol for use according to claim 11, wherein the subject is a human.

13. 1,4:3,6-dianhydrohexitol for use according to claim 11, wherein the subject is a non-human animal chosen among pets, in particular among a cat or a dog.

14. 1,4:3,6-dianhydrohexitol for use according to any the preceding claims, wherein said 1,4:3,6-dianhydrohexitol is isosorbide.
